# EUROPEAN PATENT SPECIFICATION

(11) **EP 0 570 070 B1**
(45) Date of publication and mention of the grant of the patent: **24.07.1996**
(21) Application number: 93201338.6
(22) Date of filing: 08.05.1993
(51) Int. Cl.: B01J 27/16, B01J 37/10, C07C 2/18, C07C 2/70, C08F 4/00

(54) **Solid phosphoric acid catalyst and processes for its preparation and for its use**
Fester Phosphorsäure-Katalysator, sein Herstellungsverfahren und sein Anwendungsverfahren
Catalyseur solide à l'acide phosphonique et méthodes pour sa préparation et pour son utilisation

(30) Priority: 15.05.1992 IT MI921173
(43) Date of publication of application: 18.11.1993
(73) Proprietor: SÜD-CHEMIE AG, D-80333 München (DE)
(72) Inventor: Terzoni, Giuseppe, I-29100 Piacenza (IT); Ghezzi Roberto, I-20095 Cusano Milanino, Milan (IT); Podesta' Giorgio, I-22050 Lomagna, Como (IT)
(74) Representative: Reitzner, Bruno, Dr.

(56) References cited:
- US-A- 3 661 801
- US-A- 4 912 279
- US-A- 4 946 815
- US-A- 5 043 509

## Description

The present invention relates to a process for the preparation of a catalyst for the polymerization of olefins or for the alkylation of aromatic hydrocarbons.

More specifically the present invention relates to a process for the preparation of a solid catalyst based on phosphoric acid, for the alkylation of aromatic hydrocarbons or for the polymerization of olefins, having a high resistance to hydrolysis and, therefore, a longer life of the catalyst.

The term phosphoric acid as used in the present description or in the claims, refers to one or more inorganic acids having general formula (I):

Hₙ₊₂PₙO₃ₙ₊₁ (I)

wherein n is an integer which gives a content of P₂O₅ of between 70% and 85%.

Examples of inorganic acids having general formula (I) are: orthophosphoric acid, pyrophosphoric acid, triphosphoric acid, etc.

The preferred product is a polyphosphoric acid with a P₂O₅ content equal to about 82%.

Catalysts based on phosphoric acid for the alkylation of aromatic hydrocarbons or for the polymerization of olefins are known, for example, from US patents 4.912.279 and 4.946.815.

The above patents disclose a process for the preparation of the above catalysts which includes:
- mixing an inorganic support, such as infusorial earth, diatomaceous earth, kieselguhr, clay, etc. or other minerals containing silica, with phosphoric or polyphosphoric acid with varying amounts of P₂O₅, with different ratios acid/support, at temperatures ranging from 10°C to 235°C;
- forming the material into pellets, by extrusion;
- calcining the pellets in the presence of water vapour at a temperature higher than 300°C.

The water vapour present during the calcination may be that contained in the catalyst or it may be added externally. Generally, it is considered important to add the water at the highest temperatures in order to give the finished catalyst a sufficiently high residuous acidity.

The catalysts thus obtained have the following characteristics:
- a fraction of pores with a diameter greater than 1.000 mm (10.000 Å) equal to or less than 25%; and
- a total crystallinity, obtained with respect to a standard of α-alumina, of at least 30%:

The catalysts described in the known art however have several disadvantages.

In fact, catalysts of the type described above, when used in polymerization reactions of olefins or the alkylation of aromatic hydrocarbons, must be very frequently discharged from the reactor due to their disgregation. This is favoured by the presence of water in the reagents which causes the hydrolysis of the components of the skeleton of the catalyst. This disgregation causes, as a consequence, an increase in the pressure drop in the catalytic bed.

The Applicant has now found a new process for the preparation of a solid catalyst based on phosphoric acid having a long life of the catalyst in that there are much fewer of the disadvantages of the known art.

The present invention consequently relates to a process for the preparation of a solid catalyst based on phosphoric acid comprising:
- mixing an inorganic support containing silica, with phosphoric acid;
- forming the material into pellets;
- calcining the pellets at a temperature between 400 °C and 500 °C;
characterized in that, after calcination, the catalyst is cooled to a temperature of between 80°C and 180°C and subsequently treated with a mixture of air and water vapour.

According to a preferred embodiment of the process of the present invention, the phosphoric acid is mixed with the inorganic support which is in a finely subdivided form to obtain a more homogeneous mixture and, consequently, a more compact catalyst. The diameter of the particles of the inorganic support is between 1 µm and 50 µm.

In the process of the present invention, the phosphoric acid may be used alone, or mixtures of phosphoric acid with different degrees of polymerization may be used.

Examples of inorganic supports are: infusorial earth, diatomaceous earth, kieselguhr, clay, or other porous minerals, either natural or synthetic, the preferred being those containing silica as a predominating component in quantities generally higher than 70%.

The mixing of the raw materials is carried out at temperatures ranging from 20°C to 60°C, preferably between 30°C and 50°C. Satisfactory results are obtained by heating the phosphoric acid to 60°C-80°C and mixing it subsequently with the inorganic support.

The phosphoric acid and inorganic support thus mixed form a composite wherein the weight ratio between phosphoric acid and inorganic support can vary between 2 and 3, preferably between 2.5 and 2.8 so as to have a concentration of P₂O₅ of between 55% and 61%.

The composite has a slightly humid, almost dry appearance, but it shows a plastic behaviour when subjected to pressure in a hydraulic press or extruder.

Pellets having a diameter of between 3 mm and 10 mm, preferably of about 5-6 mm are produced.

The thermal treatment (calcination) of the particles obtained by extrusion, is carried out with a gradient which is sufficient for the catalyst to reach the maximum treatment temperature in times of between 0.5 and 1.5 hours.

The maximum temperature, during the calcination, is between 400°C and 550°C, preferably between 450°C and 500°C and the permanence time in this stage is between 1 and 5 hours, preferably between 2 and 4 hours.

After the catalyst has been cooled, the subsequent treatment with air and water vapour is carried out at temperatures ranging from 80°C to 180°C, preferably between 100°C and 140°C, for a time of between 1 and 5 hours, preferably between 2 and 4 hours.

The concentration of water in the mixture air/vapour is between 5% and 100% by volume, preferably between 20% and 60% by volume.

The catalyst thus obtained is basically composed of crystalline silicon phosphates, mainly ortho [Si₃(PO₄)₄], (SOP) and pyro [SiP₂O₇], (SPP).

The catalyst obtainable by operating according to the process of the present invention, is characterized by:
- a ratio SOP/SPP in the crystalline phase, determined as a ratio between the integrated XRD reflection intensities (113 of SOP and 002 of SPP), of less than 4, preferably between 0.5 and 2.5;
- a total crystallinity, taken with respect to the reflections of an α-alumina standard (Al₂O₃), of less than 40% whereby, in particular, the reflection intensity of the SOP present is always less than 30%;
- and that the fraction of pores with a diameter greater than 1.000 mm (10.000 Å) is not less than 30%.

A certain quantity of amorphous phase may also be present in the catalyst.

The above catalyst, when used in the alkylation of aromatic hydrocarbons or polymerization of olefins, has a greater resistance to hydrolysis and consequently a much higher duration of the catalytic activity.

Treatments in other operating conditions than those specified give less resistent and/or less active catalysts.

The following examples, which are purely illustrative, provide a better understanding of the present invention and enable its embodiment.

### EXAMPLE 1

Diatomaceous earth and phosphoric acid, having a total phosphorous content expressed as P₂O₅ of 82%, are mixed in a ratio acid/diatomaceous earth equal to 2.5 p/p. The mixing is carried out at about 30°C.

The physical mixture thus obtained is fed to an extruder which presses it into pellets having a diameter of 5 mm and a length varying from 8 mm to 12 mm.

The pellets obtained have a very low mechanical resistance and are composed of amorphous phases.

Quantities of 1500 g of the material prepared according to the above process, are thermally treated in an oven with a rigorous temperature control and equipped with an air-inlet system, preheated to the temperature of the oven, and an extraction system for the vapours present in the treatment chamber.

The maximum calcination temperature, different for each quantity, is maintained for three hours and is equal to 350°C, 400°C, 450°C and 500°C. The total duration of the treatment is four hours.

In this phase only the water expelled from the particles of the catalyst is present.

Catalysts A, B, C and D are thus obtained.

The structural characteristics of the catalysts obtained are shown in Table 1.

The data indicated in Table 1 show that thermal treatment at high temperatures is necessary for forming satisfactory quantities of pyrophosphate. However catalysts are obtained with a total crystallinity, taken with respect to the reflections of an α-alumina standard (Al₂O₃), higher than 40% and with low free acidity values which influences theirs activity.

### EXAMPLE 2

Phosphoric acid and diatomaceous earth were mixed according to the process described in example 1 varying only the ratio acid/diatomaceous earth which, in this case, is equal to 2.7.

The material obtained was extruded and calcined using the same conditions described in example 1 with a maximum temperature of 450°C.

The catalyst (E) has the following characteristics:
- free acidity 3.7;
- I_{SOP}/I_{SPP} = 1.0;
- (I_{SOP}+I_{SPP}) x 100/Al₂O₃ = 41.

### EXAMPLE 3

The materials obtained using the processes described in examples 1 and 2, were treated in similar apparatus to that used for the calcination, with a mixture of air and water vapour under different operating conditions.

Table 2 shows the operating conditions and the characteristics of the materials obtained.

During the inlet of the water vapour, there is a hexothermal reaction; in all cases the maximum temperature of the catalyst reached did not exceed the set temperature by more than 10°C - 20°C.

The data indicated in Table 2 show that the air/vapour treatment, directed at restoring the free acidity, must be carried out at low temperatures to enable the ratio ortho/pyrophosphate to be within the limits previously described.

### EXAMPLE 4

Some of the catalysts, prepared as described in the previous examples, were tested in the polymerization reaction of propylene in a pilot plant.

The operating conditions were the following:

| | |
|---|---|
| - inlet temperature | 170°C |
| - pressure | 45 atm |

The composition of the feedstock was as follows:

| | |
|---|---|
| - propylene | 50% by weight |
| - propane | 50% by weight |
| - LHSV | 2.0 ml/g/hr |

The parameters taken into consideration are the initial conversion of the propylene and the duration of the catalyst expressed in hours.

For these tests the final exhaustion of the catalyst is considered as the moment when the pressure drop through the catalytic bed exceeds 5 atm.

The results thus obtained are shown in Table 3.

From the table it can be noted that catalysts A, G and L, which were calcined at low temperatures or hydrated at high temperatures and C which was not treated with air/vapour and which are therefore comparative examples, have a shorter life with respect to the catalysts of the present invention.

**TABLE 1**

| Catalyst | Temp. °C | (1) I_{SOP}/(2) I_{SPP} | (3) (I_{SOP}+I_{SPP}) x100/Al₂O₃ | (4) Free acidity |
|---|---|---|---|---|
| A | 350 | >100 | 35 | 17.2 |
| B | 400 | 7 | 40 | 10.7 |
| C | 450 | 1.2 | 47 | 3.5 |
| D | 500 | 1.0 | 47 | 2.2 |

| | | | | |
|---|---|---|---|---|
| (1) Integrated intensity of reflection 113 of SOP | | | | |
| (2) Integrated intensity of reflection 002 of SPP | | | | |
| (3) Sum of the integrated intensities compared with an α-alumina standard (Al₂O₃) | | | | |
| (4) Expressed as P₂O₅ which can undergo cold-extraction. | | | | |

**TABLE 2**

| (*) Cat. | (**) Start. Catal. | Temp. (°C) | Air/Vapor (v/v) | Time (hr) | (4) Free acidity | (1) I_{SOP}/(2) I_{SPP} | (3) (I_{SOP}+I_{SPP)} x100/Al₂O₃ |
|---|---|---|---|---|---|---|---|
| F | C | 120 | 50/50 | 2.5 | 17.6 | 1.1 | 32 |
| G | C | 200 | 50/50 | 3.5 | 16.4 | 25 | 40 |
| H | A | 120 | 80/20 | 2.0 | 23.7 | > 100 | 30 |
| I | B | 120 | 70/30 | 2.5 | 15.9 | 3.5 | 30 |
| L | D | 200 | 50/50 | 4.0 | 15.8 | 20 | 38 |
| M | D | 120 | 60/40 | 4.0 | 16.6 | 0.9 | 25 |
| N | E | 120 | 60/40 | 3.0 | 17.2 | 0.9 | 29 |

| | | | | | | | |
|---|---|---|---|---|---|---|---|
| (*) Catalyst obtained | | | | | | | |
| (**) Starting catalyst | | | | | | | |
| (1), (2), (3) and (4): see notes to Table 1 | | | | | | | |

**TABLE 3**

| Cat. | (4) free acidity | (1) I_{SOP}/(2) I_{SPP} | (3) (I_{SOP}+I_{SPP}) x100/Al₂O₃ | Prop. conver | Time (hr) |
|---|---|---|---|---|---|
| A* | 17.2 | >100 | 35 | 95 | 400 |
| F | 17.6 | 1.1 | 32 | 87 | 1200 |
| I | 15.9 | 3.5 | 30 | 84 | 800 |
| G* | 16.4 | 25 | 40 | 88 | 500 |
| L* | 15.8 | 20 | 38 | 82 | 550 |
| M | 16.6 | 0.9 | 25 | 84 | 1300 |
| N | 17.2 | 0.9 | 29 | 83 | 1250 |
| C* | 3.5 | 1.2 | 47 | < 10 | n.d. |

| | | | | | |
|---|---|---|---|---|---|
| * Catalysts A, G, L and C are comparative examples | | | | | |
| (1), (2), (3) and (4): see notes to Table 1 | | | | | |

## Claims

1. Process for the preparation of a solid catalyst based on phosphoric acid comprising:
- mixing an inorganic support containing silica, with phosphoric acid;
- forming the material into pellets;
- calcining the pellets at a temperature between 400 °C and 500 °C;
characterized in that, after calcination, the catalyst is cooled at a temperature of between 80 °C and 180 °C and subsequently treated with a mixture of air and water vapour.

2. Process according to claim 1, wherein the phosphoric acid is mixed with the inorganic support which is in a finely subdivided form.

3. Process according to claim 2, wherein the diameter of the particles of the inorganic support is between 1 µm and 50 µm.

4. Process according to claims 1, 2 or 3, wherein the inorganic supports are: infusorial earth, diatomaceous earth, kieselguhr, clay or other porous minerals, either natural or synthetic, containing silica as the predominating component in quantities generally higher than 70%.

5. Process according to the previous claims, wherein the mixing of the raw materials is carried out at temperatures ranging from 20°C to 60°C.

6. Process according to the previous claims, wherein the phosphoric acid is heated to 60°C-80°C and subsequently mixed with the inorganic support.

7. Process according to the previous claims, wherein the weight ratio between phosphoric acid and inorganic support can vary between 2 and 3 and the concentration of P₂O₅ is between 55% and 61%.

8. Process according to the previous claims, wherein the thermal treatment, calcination, of the particles obtained by extrusion, is carried out with a gradient which enables the catalyst to reach the maximum treatment temperature in times of between 0.5 and 1.5 hours.

9. Process according to the previous claims, wherein the maximum temperature, during calcination, is between 400°C and 550°C and the residence time at this stage is between 1 and 5 hours.

10. Process according to the previous claims, wherein after cooling the catalyst, the subsequent treatment with air and water vapour is carried out at temperatures ranging from 80°C to 180°C, for a period of between 1 and 5 hours.

11. Process according to the previous claims, wherein the concentration of water in the mixture air/vapour is between 5% and 60 % by volume.

12. Catalyst obtainable by the process according to any of the previous claims, composed of crystalline silicon ortophosphate, SOP, of formula Si₃(PO₄), and pyrophosphate, SPP, of formula SiP₂O₇, characterized by:
- a ratio SOP/SPP in the crystalline phase, determined as a ratio between the integrated XRD reflection intensities, 113 of SOP and 002 of SPP, of less than 4;
- a total crystallinity, taken with respect to the reflections of an α-alumina standard, Al₂O₃, of less than 40 % whereby the reflection intensity of SOP present is always less than 30 %;
- and that the fraction of pores with a diameter greater than 1000 nm (10.000 Å) is not less than 30 %.

13. Catalyst according to claim 12, wherein a further amorphous phase is present.

14. Process for the alkylation of aromatic hydrocarbons in the presence of the catalyst referred to in claims 12 and 13.

15. Process for the polymerization of olefins in the presence of the catalyst referred to in claims 12 and 13.

## Patentansprüche

1. Verfahren zur Herstellung eines festen Katalysators auf der Basis von Phosphorsäure, umfassend:
- Mischen eines anorganischen, Siliciumdioxid enthaltenden Trägers mit Phosphorsäure;
- Überführen des Materials in Pellets;
- Calcinieren der Pellets bei einer Temperatur zwischen 400°C und 500°C;
dadurch gekennzeichnet, daß der Katalysator nach dem Calcinieren auf eine Temperatur zwischen 80°C und 180°C abgekühlt und anschließend mit einem Gemisch aus Luft und Wasserdampf behandelt wird.

2. Verfahren nach Anspruch 1, worin die Phosphorsäure mit dem anorganischen Träger, der in einer feinverteilten Form vorliegt, vemischt wird.

3. Verfahren nach Anspruch 2, worin der Teilchendurchmesser des anorganischen Trägers zwischen 1 µm und 50 µm beträgt.

4. Verfahren nach Anspruch 1, 2 oder 3, worin die anorganischen Träger Infusorienerde, Diatomeenerde, Kieselgur, Ton oder andere poröse Mineralien, entweder natürlichen oder synthetischen Ursprungs, sind, die Siliciumdioxid als vorwiegende Komponente in Anteilen von im allgemeinen über 70 % enthalten.

5. Verfahren nach den vorstehenden Ansprüchen, worin das Mischen der Rohmaterialien bei Temperaturen im Bereich von 20°C bis 60°C ausgeführt wird.

6. Verfahren nach den vorstehenden Ansprüchen, worin die Phosphorsaure auf 60°C bis 80°C erwärmt und anschließend mit dem anorganischen Träger vermischt wird.

7. Verfahren nach den vorstehenden Ansprüchen, worin das Gewichtsverhältnis zwischen Phosphorsäure und anorganischem Träger zwischen 2 und 3 variieren kann und die P₂0₅-Konzentration zwischen 55 % und 61 % beträgt.

8. Verfahren nach den vorstehenden Ansprüchen, worin die Wärmebehandlung, das Calcinieren, der durch Extrusion erhaltenen Teilchen mit einem Gradienten ausgeführt wird, der den Katalysator die maximale Behandlungstemperatur in Zeiten zwischen 0,5 und 1,5 Stunden erreichen läßt.

9. Verfahren nach den vorstehenden Ansprüchen, worin die Höchsttemperatur während des Calcinierens zwischen 400°C und 550°C beträgt und die Verweilzeit in diesem Stadium zwischen 1 und 5 Stunden ausmacht.

10. Verfahren nach den vorstehenden Ansprüchen, worin nach dem Abkühlen des Katalysators die anschließende Behandlung mit Luft und Wasserdampf bei Temperaturen im Bereich von 80°C bis 180°C während einer Dauer zwischen 1 und 5 Stunden ausgeführt wird.

11. Verfahren nach den vorstehenden Ansprüchen worin die Wasserkonzentration in dem Luft/Dampfgemisch zwischen 5 % und 60 %, auf das Volumen bezogen, beträgt.

12. Nach dem Verfahren gemäß einem der vorstehenden Ansprüche erhältlicher Katalysator, bestehend aus kristallinem Siliciumorthophosphat, SOP, mit der Formel Si₃(PO₄) und Pyrophosphat, SPP, mit der Formel SiP₂0₇, gekennzeichnet durch
- ein SOP/SPP-Verhältnis in der kristallinen Phase, bestimmt als ein Verhältnis zwischen den integrierten XRD-Reflexionsintensitäten, 113 von SOP und 002 von SPP, von kleiner als 4;
- eine Gesamtkristallinität, bezogen auf die Reflexionen eines α-Aluminiumoxidstandards (Al₂0₃), von weniger als 40 %, wobei die Reflexionsintensität von vorliegendem SOP stets kleiner als 30 % ist;
und dadurch, daß die Porenfraktion mit einem Durchmesser von größer als 1000 nm (10.000 Å) nicht kleiner als 30% ist.

13. Katalysator nach Anspruch 12, worin eine zusätzliche amorphe Phase zugegen ist.

14. Verfahren zur Alkylierung von aromatischen Kohlenwasserstoffen in Anwesenheit des in den Ansprüchen 12 und 13 angesprochenen Katalysators.

15. Verfahren zur Polymerisation von Olefinen in Anwesenheit des in den Ansprüchen 12 und 13 angesprochenen Katalysators.

## Revendications

1. Procédé de préparation d'un catalyseur solide à base d'acide phosphorique comprenant :
- le mélangeage d'un support minéral contenant de la silice avec de l'acide phosphorique,
- la mise en forme de ce matériau sous forme de pastilles,
- la calcination des pastilles à une température comprise entre 400 °C et 500 °C,
caractérisé en ce que, après calcination, le catalyseur est refroidi à une température comprise entre 80 et 180 °C et est ensuite traité avec un mélange d'air et de vapeur d'eau.

2. Procédé conforme à la revendication 1 dans lequel l'acide phosphorique est mélangé avec le support minéral qui est sous une forme finement divisée.

3. Procédé conforme à la revendication 2 dans lequel le diamètre des particules de support minéral est compris entre 1 µm et 50 µm.

4. Procédé conforme à la revendication 1, 2 ou 3 dans lequel les supports minéraux sont de la terre d'infusoires, de la terre à diatomées, du gel de silice, de l'argile ou d'autres matériaux poreux naturels ou synthétiques contenant de la silice comme composant prédominant en des quantités généralement supérieures à 70 %.

5. Procédé conforme aux revendications précédentes dans lequel le mélangeage des matières premières est effectué à des températures comprises entre 20 et 60 °C.

6. Procédé conforme aux revendication précédente dans lequel l'acide phosphorique est chauffé à 60 - 80 °C et est ensuite mélangé avec le support minéral.

7. Procédé conforme aux revendication précédentes dans lequel le rapport en poids de l'acide phosphorique au support minéral peut varier entre 2 et 3 et la concentration de P₂O₅ est comprise entre 55 et 61 %.

8. Procédé conforme aux revendications précédentes dans lequel le traitement thermique, la calcination, des particules obtenues par extrusion est effectué avec un gradient qui permet au catalyseur d'atteindre la température maximale de traitement en un temps compris entre 0,5 et 1,5 heure.

9. Procédé conforme aux revendications précédentes dans lequel la température maximale pendant la calcination est comprise entre 400 et 550 °C et le temps de séjour dans cette étape est compris entre 1 et 5 heures.

10. Procédé conforme aux revendications précédentes dans lequel, après refroidissement du catalyseur, le traitement suivant par de l'air et de la vapeur d'eau est effectué à des températures comprises entre 80 et 180 °C, pendant une durée comprise entre 1 et 5 heures.

11. Procédé conforme aux revendications précédentes dans lequel la concentration de l'eau dans le mélange air/vapeur est comprise entre 5 et 60 % en volume.

12. Catalyseur pouvant être obtenu par le procédé conforme à une quelconque des revendications précédentes, composé d'orthophosphate de silicium cristallin (OPS) de formule Si₃(PO₄), et de pyrophosphate de silicium (PPS) de formule SiP₂O₇, caractérisé par
- un rapport OPS/PPS de la phase cristalline, déterminé comme étant le rapport des intensités de réflexion intégrées de diffraction de rayons X, 113 pour le OPS et 002 pour le PPS, inférieur à 4,
- une cristallinité totale, déterminée par rapport aux réflexions d'une α-alumine standard, Al₂O₃, inférieure à 40 %, l'intensité de réflexion du OPS présent étant ainsi toujours inférieure à 30 %, et
- une fraction de pores ayant un diamètre supérieur à 1000 nm (10 000 Å) inférieure à 30 %.

13. Catalyseur conforme à la revendication 12 dans lequel une autre phase amorphe est présente.

14. Procédé d'alkylation d'hydrocarbures aromatiques en présence du catalyseur défini dans les revendications 12 et 13.

15. Procédé de polymérisation d'oléfines en présence du catalyseur défini dans les revendications 12 et 13.
